# FASCICULE DE BREVET EUROPEEN

(11) **EP 2 280 061 B1**
(45) Date de publication et mention de la délivrance du brevet: **02.11.2011**
(21) Numéro de dépôt: 09425302.8
(22) Date de dépôt: 29.07.2009
(51) Int. Cl.: C12M 1/12, C12P 7/56

(54) **Installation et procédé pour la fermentation du sérum de lait destiné à la production d' acide lactique, ferments et bio-protéines**
Anlage und Verfahren zur Fermentation von Milchserum zur Herstellung von Milchsäure, Fermenten und Bio-Proteinen
Installation and process for the fermentation of milk serum for producing lactic acid, ferments and bio-proteins

(43) Date de publication de la demande: 02.02.2011
(73) Titulaire: Ars Nova sas di Franca Pipolo & C., 84091 Battipaglia (IT)
(72) Inventeur: Fortunato, Vittorio, 84091 Battipaglia (SA) (IT); Fortunato, Sabato, 84091 Battipaglia (SA) (IT); Fortunato, Luca, 84091 Battipaglia (SA) (IT)

(56) Documents cités:
- EP-A- 0 265 409
- EP-A- 0 657 542
- DE-A1- 3 049 381
- DE-C1- 19 580 599
- FR-A- 2 555 200
- JP-A- 9 193 090
- US-A- 5 746 920

## Description

### Description

L'objet de la présente invention est l'installation et le relatif procédé de transformation du sérum de lait, dérivant de la fabrication industrielle de fromages à pâte filée, en particulier de la rigotte, en acide lactique à destiner au liquide de gouvernement des produits laitiers, en substrat recyclé pour la fermentation et en bio-protéines à destiner à l'alimentation zootechnique ou aux fromages pro-biotiques. Le traitement du sérum et des autres effluents des produits fromagers pour en réduire les taux de pollution représente, pour les entreprises de produits laitiers italiennes, qui sont presque toujours de taille moyenne-petite, un des problèmes majeurs, là où il est impossible de procéder à leur élimination à bas coût.

L'utilisation directe dans l'alimentation zootechnique n'est une pratique avantageuse que dans les situations où l'élevage est proche, ou presque, à la fromagerie même, et ne constitue pas une solution proposable dans la plupart des cas.

Parmi les alternatives possibles pour la mise en valeur des effluents de fromagerie, parmi celles qui se sont montrées susceptibles d'une mise en place effective dans le contexte national, on retrouve l'utilisation du sérum pour la production fermentative à bas coût d'acide lactique ou de bio-protéines (levures) à destiner à l'alimentation zootechnique ; en effet, parmi les différents produits qu'on peut obtenir de la fermentation du lactose contenu dans les effluents des produits fromagers, un des produits les plus intéressants, en raison du grand nombre d'applications possibles, est sans doute l'acide lactique, qui est utilisé en plusieurs secteurs industriels, à partir de l'industrie alimentaire, pour arriver à l'industrie cosmétique, au nettoyage et à l'hygiène personnelle (étant donné que le pH de l'acide lactique est le même que celui du tissu épithélial humain), jusqu'à l'utilisation comme réactif spécifique pour certaines opérations spécifiques (dégraissage, décapage et cetera) dans l'industrie manufacturière.

Un procédé de production d'acide lactique par fermentation de lactoserom est décrit dans la demande européenne EP 0 265 409. Ce procédé comporte une étape d'ultrafiltration du moût de fermentation.

Actuellement, la méthode classique pour la production de l'acide lactique utilise comme substrat de fermentation les sous-produits (mélasse) de l'industrie sucrière, alors que dans la présente invention on propose un procédé et la relative installation capables d'utiliser le sérum de lait en tant que substrat, qui peut être transformé en acide lactique avec un rendement supérieur à 50% sur la base de la substance sèche total. Le procédé proposé nous permet de mettre en valeur le sérum de lait dérivant de la fabrication de la rigotte par sa transformation en acide lactique d'application rapide, puisque les fromageries nécessitent l'acide lactique pour préparer la « petite sauce » ou le liquide de gouvernement des produits laitiers à pate filée, tels que la mozzarella, la scamorza, etc., ou bien de les utiliser comme substrats à bas coût pour des procédés fermentatifs, et pour la transformation du sérum en acide lactique, et pour l'obtention de biomasses microbiennes (par exemple, les levures du type Kluyveromyces, déjà autorisées par les organes compétents de l'EU pour la formulation des aliments), avec un haut contenu protéique et vitaminique (bio-protéines) à destiner à la formulation d'aliments pour les élevages zootechniques ou les fromages pro-biotiques.

Le procédé de base est bien connu et consiste essentiellement dans une première phase de séparation et récupération des protéines du sérum (jusqu'au-delà de 75% au total) et dans la fermentation subséquente de la solution de lactose au 3-4% pour obtenir les levures sous forme d'une poussière avec un contenu d'humidité inférieur à 5%, prêtes pour être confectionnées et commercialisées. L'objectif donc de la présent invention est de proposer un procédé et une installation qui utilisent le sérum dérivant ou provenant de la fabrication de rigotte pour produire, dans des conditions contrôlées, l'acide lactique obtenu de la fermentation du lactose et par la sédimentation, une fois que la fermentation s'est produite, de protéines réutilisables pour la production de fromages pro-biotiques ou d'aliments pour les animaux.

Un autre objectif de la présente invention, en accord avec l'objectif précédent, est de proposer un procédé et une installation qui utilisent les bactéries lactiques de type alimentaire (yaourt) pour fermenter le lactose contenu dans le sérum du lait.

Un autre objectif de la présente invention, en accord avec l'objectif précédent, est de proposer une installation constitue par : un containeur en acier à double paroi thermostat et automatisé, là où se produit la fermentation contrôlée, la production et la collecte de l'acide lactique réutilisable en tant que liquide de gouvernement des produits laitiers, et en forme d'entonnoir dans la partie basse pour obtenir un décanteur d'où est pris le sédiment pour la formulation d'aliments ou fromages pro-biotiques, ultérieurement relié à un réservoir frigo où le contenu (ferments) est destiné au recyclage pour la production d'acide lactique.

Un autre objectif de la présente invention, en accord avec l'objectif précédent, est de proposer un procédé et une installation complètement automatisés qui permettent d'obtenir une quantité programmée d'acide lactique selon la durée de la fermentation, ainsi que la production d'acide acétique et d'autres produits dérivant de la fermentation du lactose en variant les conditions d'oxygène, température et ferments.

Un autre objectif de la présente invention, en accord avec l'objectif précédent, est de proposer un procédé et une installation dans laquelle le produit liquide fermenté, filtré à 0.1 micron, peut être facilement traité par des procédés à membrane pour concentrer l'acide lactique en ayant enlevé les protéines et le lactose.

Un autre objectif de la présente invention, en accord avec l'objectif précédent, est celui de proposer un procédé et une installation dans laquelle le fermenté liquide en excès, c'est-à-dire celui non utilisé, peut être facilement dépuré dans un dépurateur biologique des boues activées, parce qu'il a perdu le lactose et les protéines.

Celles-ci et d'autres caractéristiques, ainsi que les avantages, sont évidents dans la description suivante et dans le dessein annexé, qui est fourni seulement à titre indicatif et non limitatif, dans lequel :

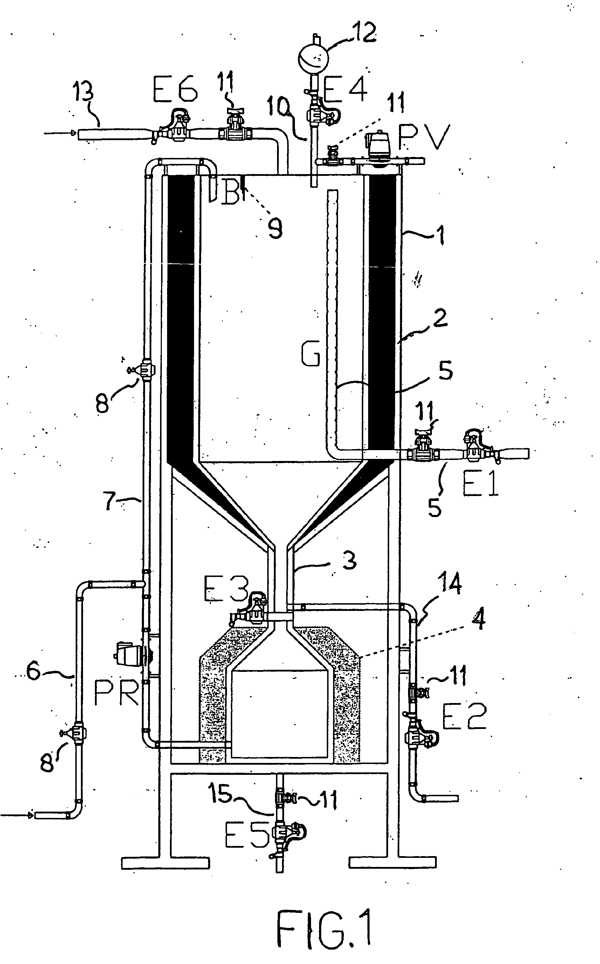

la figure 1 montre, dans une vue schématique, l'installation pour la fermentation du sérum de lait en acide lactique et la récupération des bio-proteines.

Conformément au dessin attaché, on peut afficher une installation pour la fermentation constituée par un containeur cylindrique (1) en acier inox avec un fond conique (dénommé brièvement réacteur), à double parois (2) avec circulation de l'eau dans la parois intermédiaire pour assurer une température contrôlée à l'intérieur du cylindre ; sur le fond du cône, un tube (3) relie un réservoir (4) cylindrique réfrigerable, dont les dimensions sont d'environs 1/30 par rapport à la capacité du containeur cylindrique supérieur (1) (réacteur). Un dispositif à station thermique assure le contrôle de la température, et la température intérieure au réacteur, variable de 38 à 45°C, et la température du réservoir qui accumule les sédiments (ferments) réfrigérés (4), variable entre 2° ou 4°C, et en plus le contrôle de la température du sérum en entrée et du bain thermostatique du réservoir. Le dispositif pour le déchargement du produit fermenté est constitué par un tuyau en acier inox, G, percé (5) le long de sa superficie, placé verticalement dans le réacteur et actionné par une électrovanne temporisée E1 et une soupape à bille posée sur le terminal extérieur (5 ') du tuyau (5). Le déchargement se produit après la fermeture de l'électrovanne E3 posée sur le tuyau (3) qui isole le réservoir (4) et donc les sédiments (ferments) qu'il contient.

Le sérum dérivant de la fabrication de rigotte est introduit par la tuyauterie (6), il se relie ensuite à la tuyauterie (7) qui part du réservoir (4) pour rejoindre le sommet du réacteur (1). Les tuyauteries (6) et (7) sont pourvues de soupapes d'arrêt tout au long de leur parcours (8). Toutes les tuyauteries sont pourvues d'électrovannes, même de soupapes à bille (11). La tuyauterie (7) est pourvue d'une pompe PR qui mélange les ferments et le matériel cumulé dans le réservoir frigo (4) avec le sérum dérivant de la fabrication de rigotte à l'entrée de la tuyauterie (6). Sur le sommet du réacteur (1) sont positionnées une sonde de niveau (9) pour régler le remplissage, une canalisation (10) pourvue d'une soupape à bille (11) pour une pompe PV pour extraire l'air et créer le vide à l'intérieur du réacteur et une électrovanne E4 avec le filtre (12) à 0.1 micron pour filtrer l'air à l'entrée. Une ultérieure canalisation (13) avec une electrovanne E6 et une soupape à bille (11) permet l'entrée de vapeur à 120 °C pour le lavage du réacteur. Le déchargement de la fraction de produit fermenté, mélangé à des sédiments qui ne sont pas rentrés dans le réservoir (4), se produit par la tuyauterie (14) et l'ouverture de l'électrovanne E2. La tuyauterie (15) par l'électrovanne E5 sert à décharger automatiquement, après un discret nombre de cycles, le produit en excès ou à rénover la charge de ferments lactiques ou à se nettoyer par elle-même.

### Description du procédé pour la fermentation du sérum de lait.

Le sérum de rigotte à l'entrée de la tuyauterie (6) est préalablement mélangé par l'amorçage, constitué par le ferment lactique ou le mélange de ferments préchoisis, à une température inférieure à celle de fermentation. Toutes les électrovannes sont fermées, sauf l'électrovanne E3, alors qu'en même temps la pompe à vide PV reprend à fonctionner (pour évacuer l'oxygène du réacteur afin d'éviter des fermentations alternatives, puisque les conditions de production de l'acide lactique sont anaérobiques, c'est-à-dire sans oxygène; en effet, la présence d'oxygène pourrait causer la production d'autres acides, parmi lesquels l'acide acétique) ; le remplissage du réacteur en sérum s'arrête lorsque la sonde de niveau B émet le signal complet et la pompe à vide s'arrête en même temps. Dans la phase suivante, le thermostat contrôle que dans la double paroi (2) la température demandée pour la fermentation du lactose avec les bactéries lactiques soit atteinte et maintenue, c'est-à-dire une température entre 38°C et 45°C selon le lactobacille utilisé, jusqu'à la fin de la phase de fermentation (dont la durée temporelle est, comme l'on a déjà dit, variable, et peut être de 6 heures, ou bien de 8 heures, etcetera), le réservoir frigo reste isolé pour la fermeture de l'électrovanne E3. À la fin de la phase de fermentation on a l'ouverture de l'électrovanne E4 avec le filtre à air à 0.1 micron pour empêcher l'entrée de poussières et de microorganismes (puisque la fermentation doit se produire dans un environnement stérile pour éviter des contaminations et par conséquent des fermentations alternatives), et l'ouverture de l'électrovanne E1 et la fermeture de l'électrovanne E3, de sorte que il puisse se produire le déchargement du produit fermenté, de l'acide lactique, alors que la partie sédimentable constituée par des protéines, polysaccarides polymérisés, ferments lactiques et enzymes reste piégée dans le réservoir frigo qui peut s'activer en portant la température à son intérieur à 2-4°C.

Un éventuel excès du produit fermenté et mélangé à des sédiments peut être ramassé dans un autre récipient. La phase suivante peut être aussi le lavage à l'intérieur du réacteur avec l'entrée de vapeur à 120 °C, une fois que l'électrovanne de lavage E6 s'est ouverte.

Dans le cycle de fermentation suivant on a l'entrée du sérum à une température inférieure à la température de fermentation, et en même temps l'ouverture de l'électrovanne E3 et l'actionnement de la pompe PR qui porte les ferments en cycle dans la tuyauterie (7) et mélange les ferments et le matériel cumulé dans le réservoir frigo avec le sérum dérivant de la fabrication de rigotte à l'entrée de la tuyauterie (6). Toutes les autres électrovannes sont fermées pendant que la pompe à vide PV est en action ; les phases précitées se suivent.

Le cycle est réglé par l'armoire électrique avec PLC qui règle les phases de fonctionnement avec l'actionnement de pompes, électrovannes, station thermique et ph mesurés avec des équipements faciles à repérer et à installer, par la programmation et selon les caractéristiques demandées.

L'évolution de la fermentation peut être suivie facilement en contrôlant le ph ainsi qu'en contrôlant la teneur d'acide lactique produit ; on peut donc obtenir un produit avec la concentration d'acide lactique souhaitée simplement en réglant le temps de fermentation. Si une partie de sérum avec un ph = 5,6 est injecté avec des ferments lactiques, après 6 heures nous avons un ph = 4,2 et après 12 heures nous aurons un ph =3.8 en raison essentiellement de la transformation du sérum en acide lactique (après 24 heures le ph = 3,4).

De la fermentation lactique on obtient l'acide lactique qui est un liquide jaune clair opalescent avec des petites quantités de matériels suspendues qui peuvent être enlevées facilement par filtration, par ex. à 0,1 micron et donc privées de bactéries, levures et moisissures et intégralement réutilisables comme liquide de gouvernement des produits laitiers, éventuellement pasteurisé et dans ce cas la filtration, qui peut même être évitée.

Entretemps, seulement après 2-3 heures de fermentation, nous aurons la coagulation des protéines qui sédimentent sur le fond du réacteur dans le réservoir frigo approprié afin d'être conservées et réutilisées pour l'injection des fermentations suivantes, puisqu'elles sont riches de ferments lactiques et enzymes, alors que les protéines en excès, produites après un discret nombre de cycles, sont déchargées automatiquement et entamées à la production de fromages pro-biotiques ou des aliments pour animaux.

La technique décrite est appliquée au sérum dérivant ou provenant de la fabrication de rigotte, afin de l'utiliser dans le sérum vierge, qui est plus riche en gras et protéines. Ceci doit être prétraité par centrifugation, pasteurisation et filtration, écrémée et privée des protéines qui grumèlent pendant le procédé.

D'après ce qui a été décrit et illustré plus haut, on peut voir que l'invention atteint les buts préposés, en particulier elle propose un procédé économiquement intéressant, technologiquement efficace et compatible avec l'environnement, et en outre le procédé est compatible avec le monitorage de la fermentation afin de maintenir la stérilité à l'intérieur du réacteur en réduisant les interférences de l'extérieur dûes, par exemple, aux dispositifs de prélèvement par échantillon ; et, le dernier mais pas le moindre, d'économiser les coûts liés aux réactifs et à l'intervention de personnel qualifié.

## Revendications

1. Installation pour la fermentation du lactose contenu dans le sérum dérivant ou provenant de la fabrication de rigotte pour produire, en conditions contrôlées, l'acide lactique utilisable comme liquide de gouvernement des fromages à pâte filée, et par la sédimentation, une fois que la fermentation s'est produite, de protéines réutilisables pour la production de fromages pro-biotiques ou d'aliments pour les animaux, **caractérisée par le fait que** l'installation est constituée par un :
containeur (1) en acier à double parois où circule l'eau, où se produit la fermentation contrôlée par un thermostat, à une température variable entre 38 et 45°C, ainsi que la production et la collecte de l'acide lactique à travers le dispositif de déchargement constitué par un tuyau en acier inox, G, percé (5) le long de sa superficie, placé verticalement dans le réacteur et
actionné par une électrovanne temporisée E1 positionnée sur le terminal extérieur (5 ') du tuyau (5) ; le containeur (1) a une forme conique dans la partie basse pour réaliser un décanteur d'où est pris le sédiment constitué par les ferments et les bio-protéines récupérables à travers la tuyauterie (14) et
l'ouverture de l'électrovanne E2.

2. Installation pour la fermentation du lactose contenu dans le sérum dérivant ou provenant de la fabrication de rigotte selon la revendication 1), **caractérisée en ce que** l'installation en outre est formée avec un tube (3) sur le fond du cône que relie un réservoir (4) cylindrique réfrigerable, dont les dimensions sont d'environ 1/30 par rapport à la capacité du containeur cylindrique supérieur (1).

3. installation pour la fermentation du lactose contenu dans le sérum dérivant ou provenant de la fabrication de rigotte selon la revendication 1), **caractérisée e**n ce que l'installation est en outre formée par la tuyauterie (6) par laquelle est introduit le sérum dérivant de la fabrication de rigotte, il se relie ensuite à la tuyauterie (7) qui part du réservoir (4) pour rejoindre le sommet du réacteur (1).

4. Installation pour la fermentation du lactose contenu dans.le sérum dérivant ou provenant de la fabrication de rigotte selon la revendication 1), **caractérisée en ce que** l'installation est en outre formée par une pompe PR qui mélange les ferments et le matériel cumulé dans le réservoir frigo (4) avec le sérum dérivant de la fabrication de rigotte à l'entrée de la tuyauterie (6) ; les ferments et le matériel cumulé en excès sont déchargés automatiquement, via la tuyauterie (15) par l'électrovanne E5.

5. Installation pour la fermentation du lactose contenu dans le sérum dérivant ou provenant de la fabrication de rigotte selon la revendication 1), **caractérisée en ce que** sur le sommet du réacteur (1) est positionnée une sonde de niveau (9) pour régler le remplissage.

6. Installation pour la fermentation du lactose contenu dans le sérum dérivant ou provenant de la fabrication de rigotte selon la revendication 1), **caractérisée en ce que** l'installation est en outre formée par une canalisation (10) pourvue d'une soupape à bille (11) pour une pompe PV pour extraire l'air et créer le vide à l'intérieur du réacteur, par une électrovanne E4 et par le filtre (12) à 0.1 micron pour filtrer l'air à l'entrée.

7. Installation pour la fermentation du lactose contenu dans le sérum dérivant ou provenant de la fabrication de rigotte selon la revendication 1), **caractérisée en ce que** l'installation est en outre formée par une ultérieure canalisation (13) avec one électrovanne E6 et une soupape bille. (11) permet l'entrée de vapeur à 120 °C pour le lavage du réacteur.

8. Procédé pour la fermentation du lactose contenu dans le sérum dérivant ou provenant de la fabrication de rigotte, **caractérisée en ce que** le sérum de rigotte à l'entrée de la tuyauterie (6) est préalablement mélangé par le ferment lactique ou le mélange de ferments préchoisis, à une température inférieure à celle de la fermentation ; toutes les électrovannes sont fermées, sauf l'électrovanne E3, alors qu'en même temps la pompe à vide PV reprend à fonctionner pour évacuer l'oxygène du réacteur ; le remplissage du réacteur en sérum s'arrête lorsque la sonde de niveau B émet le signal complet et la pompe à vide s'arrête en même temps ; dans la phase suivante, le thermostat contrôle que dans la double parois (2) la température demandée pour la fermentation du lactose avec les bactéries lactiques soit atteinte et maintenue, c'est-à-dire une température entre 38°C et 45°C selon le lactobacille utilisée jusqu'à la fin de la phase de fermentation, le réservoir frigo reste isolé pour la fermeture de l'électrovanne E3 ; à la fin de la phase de fermentation on a l'ouverture de l'électrovanne E4 avec le filtre à air à 0.1 micron pour empêcher l'entrée de poussières et de microorganismes, et l'ouverture de l'électrovanne E1 et la fermeture de l'électrovanne E3, pour le déchargement du produit fermenté, de l'acide lactique, alors que la partie sédimentable constituée par des protéines, polysaccaride polymérisés, ferments lactiques et enzymes reste piégée dans le réservoir frigo à température 2-4°C ; un érentoel excès du produit fermenté et mélangé à des sédiments peur être ramassé dans un autre récipient ; la phase de lavage à l'intérieur du réacteur avec l'entrée de vapeur à 120 °C, une fois que l'électrovanne.de lavage E6 s'est ouverte ; dans le cycle de fermentation suivant on a l'entrée du sérum à une température inférieure à la température de fermentation, et en même temps l'ouverture de l'électro.vanne E3 et l'actionnement de la pompe PR qui porte les ferments en cycle dans la tuyauterie (7) et mélange les ferments et le matériel cumulé dans le réservoir frigo avec le sérum dérivant de la fabrication de rigotte à l'entrée de la tuyauterie (6) ; toutes les autres électrovannes sont fermées pendant que la pompe à vide PV est en action ; les phases précitées se suivent.

9. Procédé pour la fermentation du lactose contenu dans l'e sérum dérivant ou provenant de la fabrication de rigotte selon la revendication 8), **caractérisée en ce que** en variant les conditions d'oxygène, température et ferments, on obtient une quantité programmée d'acide lactique ou de lácide
acétique et d'autres produits dérivant de la fermentation du lactose

10. Procédé pour la fermentation du lactose contenu dans le sérum dérivant ou provenant de la fabrication de rigotte selon la revendication 8), **caractérisé en ce que** le produit liquide fermenté, filtré à 0.1 micron, est traité par des procédés à membrane pour concentrer l'acide lactique en ayant enlevé les protéines et le lactose, et dans laquelle le fermenté liquide en excès, c'est-à-dire celui non utilisé, peut être facilement dépuré dans un dépurateur biologique des boues activées.

## Claims

1. Installation for the fermentation of lactose contained in the serum derived or from ricotta manufacturing to produce, under controlled conditions, the lactic acid used as a preservation liquid of pulled-curd cheese, and through sedimentation, once fermentation has occurred, of reusable proteins for the production of probiotics cheese or for animal feeding stuff, **characterized in that** the insta,lation consists of a: double wall steel container (1) where water runs, where occurs fermentation which is controlled by a thermostat at a temperature varying between 38 and 45 °C, and the production and collection of lactic acid through the discharge device consisting of a stainless steel pipe, G, drilled (5) along its surface, placed vertically in the reactor and activated by a timed solenoid valve E1 placed on the outside terminal (5 ') of the pipe (5), the container (1) has a conical shape in the lower part so as to form a decanter where the sediment made up by ferments and by-proteins are recoverable through the pipe (14) and the opening of the solenoid valve E2.

2. Installation for the fermentation of lactose contained in the serum derived or from ricotta manufacturing according to claim 1), **characterized in that** the installation is also formed with a hose (3) on the bottom of the cone that connects a cooling cylindrical tank (4), whose dimensions are of about 1/30 compared to the capacity of the upper cylindrical container (1)

3. Installation for the fermentation of lactose contained in the serum derived or from ricotta manufacturing according to claim 1), **characterized in that** the installation is also formed by the pipe (6) through which the serum derived from ricotta manufacturing is also introduced, then it connects to the pipe (7) which starts from the tank (4) to reach the top of the reactor (1).

4. Installation for the fermentation of lactose contained in the serum derived or from ricotta manufacturing according to claim 1), **characterized in that** the installation is also formed by a PR pump which mixes the ferments and materials accumulated in the cooling tank (4) with the serum derived from ricotta manufacturing at the pipe entrance (6), the ferments and the material accumulated in excess are automatically discharged through the pipe (15) by the solenoid valve E5.

5. Installation for the fermentation of lactose contained in the serum derived or from ricotta manufacturing according to claim 1), **characterized in that** on the top of the reactor (1) a level sensor (9) is positioned to adjust the filling.

6. Installation for the fermentation of lactose contained in the serum derived or from ricotta manufacturing according to claim 1), **characterized in that** the plant is also formed by a conduit (10) provide with a ball valve (11) for a PV pump tb extract the air and create a vacuum inside the reactor, and by a solenoid valve E4 through the filter (12) to 0,1 micron to filter the air at the entrance.

7. Installation for the fermentation of lactose contained in the serum derived or from ricotta manufacturing according to claim 1), in which the installation is also formed by an additional conduit (13) with a solenoid valve E6 and a ball valve (11) which allows the entry of steam at 120 °C for washing the engine.

8. Method for the fermentation of lactose contained in the serum derived or from ricotta manufacturing, **characterized in that** the serum of ricotta at the pipe entrance (6) is previously mixed with the lactic ferment or with a mixture of preselected ferments at a temperature lower than that of fermentation, all valves are closed except the solenoid valve E3, while at the same time the vacuum pump PV resumes operation to remove oxygen from the reactor; the filling of the reactor stops when the serum level sensor B gives the complete signal and the vacuum pump stops at the same time: in the next stage, the thermostat monitors that in the double wall (2) the required temperature for the fermentation of lactose with the lactic acid bacteria is achieved and maintained, that is to say at a temperature between 38 ° C and 45 ° C according to the lactobacillus used until the end of the fermentation phase, the fridge tank remains isolated for closing the ball valve E3, at the end of the fermentation phase there is the opening of the solenoid valve E4 with the air filter at 0,1 micron to prevent the entry of dust and microorganisms, and the opening of the solenoid valve E1 and the closing of the solenoid valve E3, to discharge the fermented product of lactic acid, while the sedimentable part consisting of proteins, polysaccharide polymers, lactic acid bacteria and enzymes remains trapped in the cooling tank at a temperature between 2-4 ° C: any excess of the fermented product which is mixed with sediments can be collected into another container, the washing stage inside the reactor with the steam inlet at 120 ° C, once the washing solenoid valve E6 is opened, and in the next fermentation cycle the serum is entered at a temperature below the temperature of fermentation, and the simultaneous opening of the solenoid valve E3 and the operation of the PR pump which cycle the ferments through the pipe (7) and mixes the ferments and the materials accumulated in the cooling tank with the serum derived from ricotta manufacturing at the pipe entrance (6), all other solenoid valves are closed while the PV vacuum pump is operating; the above stages follow each other.

9. Method for the fermentation of lactose contained in the serum derived or from ricotta manufacturing according to claim 1) and 8), in which by varying the conditions of oxygen, temperature and ferments, a planned amount of lactic acid or acetic acid and other products derived from the fermentation of lactose may be obtained.

10. Method for the fermentations of lactose contained in the serum or derived from ricotta manufacturing according to claim 8), wherein the fermented liquid, filtered to 0.1 micron, is treated by membrane processes for concentrating the lactic acid having removed the proteins and lactose, and in which the fermented excess liquid, i.e. the liquid which is not used, can be easily depurated in a biological purifier of activated sludge

## Patentansprüche

1. Anlage zu Fermentierung von Laktose, die im Serum aus der Herstellung von Ricotta enthalten ist, unter kontrollierten Bedingungen, die Milchsäure wird als Konservierungsftüssigkeit von Pasta filata. Käse verwendet, und durch Sedimentation, nachdem die Fermentierung abgeschlossen ist, von wiederverwendbaren Proteinen zur Herstellung von probiotischem Käse bzw. Tierfutter, **dadurch gekennzeichnet, dass** die Anlage aus Folgendem besteht: doppelwandiger Stahlbehälter (1) durch den Wasser läuft, in dem Fermentierung auftritt, der durch einen Thermostat gesteuert ist, mit Temperaturen, die zwischen 38 und 45 °C schwanken, und der Erzeugung und Sammlung von Milchsäure durch das Auslassgerät, bestehend aus einem Edelstahlrohr, G, mit Bohrungen (5) entlang seiner Oberfläche, senkrecht im Reaktor platziert und über ein zeitgesteuertes Magnetventil E1 am Außenanschluss (5') des Rohrs (5), betätigt, der Behälter (1) ist im unteres Teil konisch geformt, damit sich ein Absetzgefäß ausbildet, über das die Sedimente aus den Fermenten und Bio-Proteinen durch das Rohr (14) und das geöffnete Magnetventil E2 gewonnen werden können.

2. Anlage zur Fermentierung der Laktose im Serum, das bei der Herstellung von Ricotta anfällt, gemäß Anspruch 1), **dadurch gekennzeichnet, dass** die Anlage auch über einen Schlauch (3) auf dem Boden des Konus verfügt, der mit einem zylindrischen Kühlbehälter (4) verbunden ist, dessen Abmessungen etwa. 1/30 des inhalts des oberen zylindrischen Behälters (1) entsprechen.

3. Anlage zur Fermentierung der Laktose im Serum, das bei der Herstellung von Ricotta anfällt gemäß Anspruch 1), **dadurch gekennzeichnet, dass** die Anlage auch über ein Rohr (6) verfügt, durch das das Serum, das bei der HersteXung von Ricotta anfällt, auch eingeführt wird, dann ist es mit Rohr (7) verbunden, das vom Behälter (4) bis oben auf den Reaktor verläuft (1).

4. Anlage zur Fermentierung der Laktose im Serum, das bei der Herstellung von Ricotta anfällt, gemäß Anspruch 1), **dadurch. gekennzeichnet, dass** die Anlage.auch eine PV-Pumpe enthält, die die Fermente und das im Kühlbehälter(4) angesammelte Material mit dem Serum, das bei der Herstellung von Ricotta anfäitt, am Rohreingang (6) mischt, die Fermente und das überschüssige angesammelte Material werden automatisch von Magnetventil E5 durch Rohr (15) abgelassen.

5. Anlage zur Fermentierung der Laktose im Serum, das bei der Herstellung von Ricotta anfällt, gemäß Anspruch 1) **dadurch gekennzeichnet, dass** sich oben auf dem Reaktor (1) ein Füllstandsfühler (9) befindet, über den der füllstand geregelt

6. Anlage zur Fermentierung der Laktose im Serum, das bei der Herstellung von Ricotta anfällö, gemäß Anspruch 1), **dadurch gekennzeichnet, dass** die Anlage auch einen Kanal (10) mit einem Kugelventil (11) für ein PV-Pumpe beinhaltet, um Luft aus dem Reaktor zu saugen und damit ein Vakuum herzustellen, und durch ein Magnetventil E4 durch den Filter (12) auf 0.1 μm, zur Filterung der Luft am Eingang.

7. Anlage zur Fermentierung der Laktose im Serum, das bei der Herstellung von Ricotta anfällt, gemäß Anspruch 1), in der die Anlage auch aus einem zusätzlichen Kanal (13) mit einem Magnetventil E6 und einem Kugelventil (11) besteht, die den Eintritt von 120 °C heiße Dampf zum Waschen des Reaktors gestattet.

8. Verfahren für die Fermentierung der in dem Serum, das bei der Herstellung von Ricotta anfällt, enthaltenen Laktose, **dadurch gekennzeichnet, dass** das Serum aus Ricotta am Rohreingang (6) zuvor mit dem Milchferment oder einer Mischung vorgegebener Fermente bei einer Temperatur unter der der Fermentierung gem.ischt wurde, alle Ventile sind geschlossen außer Magnetventil E3, und gleichzeitig nimmt die Vakuumpumpe Pumpe PV den Betrieb auf, um dem Reaktor Sauerstoff zu entziehen; die Befüllung des Reaktors endet, wenn der Serumfüllstandsfühler B das Signal, voll' gibt und die Vakuumpumpe hält gleichzeitig an: Im nächsten Stadium überwacht der Thermostat, dass in der
Doppelwand (2) die erforderliche Temperatur zur Fermentierung von Milchsäurebakterien erreicht und aufrecht erhalten wird, d.h. eine Temperatur zwischen 38 °C und 45 ° C je nach verwendetem Laktobakterium, bis zum Ende der Fermentierungsphase bleibt der Kühlbehälter getrennt, durch Schließen von Kugelventil E3. Am Ende der Fermentierungsphase wird Magnetventil E4 geöffnet mit dem auf 0,1 μm eingestellten Filter, um das Eindringen von Staub und Mikroorganismen zu verhindern, und das Öffnen von Magnetventil E1 und das Schließen von Magnetventil E3 führen zum Ablassen des Fermentationsprodukts der Milchsäure, wohingegen der sedimentierbare Teil bestehend aus Proteinen, polimerisierten Polisacchariden, Milchsäurebakterien und Enzymen im Kühlbehälter bei einer Temperatur zwischen 2 und 4 °C gefangen bleiben: Das überschüssige fermentierte Produkt, das mit den Sedimenten gemischt ist, kann in einem anderen Behälter aufgefangen werden, die Waschphase im Reaktor, bei der der Dampf mit 120 ° C eintritt, sobald das Waschmagnetventil E6 geöffnet wird, und im nächsten Fermentierungszyklus tritt das Serum bei einer Temperatur unter der Fermentierungstemperatur ein, und gleichzeitig öffnen das Magrietventil E3 und die PR Pumpe, die die Fermente durch Rohr (7) pumpt und die Fermente und die im Kühlbehälter angesammelten Materialien mit dem Serum, das bei der Herstellung von , Ricotta anfällt, am Rohreingang (6) mischt, nimmt den Betrieb auf; all anderen Magnetventile sind geschlossen, während die PV-Vakuumpumpe läuft; die og. Phasen laufen nach einander ab.

9. Verfahren für die Fermehtierung der in dem Serum, das bei der Herstellung von Ricotta anfällt, enthattenen Laktose, gemäß Anspruch 1) und 8), in dem durch unterschiedliche Umgebungsbedingungen wie Sauerstoffgehalt, Temperatur und Fermente vorgegebene Mengen Milchsäure, Essigsäure und andere Erzeugnisse aus der Fermentierung von Laktose gewonnen werden können.

10. Verfahren für die Fermentierung der in dem Serum, das bei der Herstellung von Ricotta anfällt, enthaltenen Laktose, gemäß Anspruch 8), wobei die fermentierte Flüssigkeit, auf 0,1 μm ausgefiltert, mittels Membranverfahren zur Konzentrierung der Milchsäure und durch Entfernen der Milchsäure und Laktose aufbereitet werden kann, und bei dem die überschüssige fermentierte Flüssigkeit, d h. die Flüssigkeit, die nicht verwendet wird, leicht in einem biologischen Reinigungsgerät von Belebtschlamm befreit werden kann.
